# EUROPEAN PATENT APPLICATION

(11) **EP 4 339 617 A1**
(43) Date of publication of application: **20.03.2024**
(21) Application number: 22807242.7
(22) Date of filing: 25.03.2022
(51) Int. Cl.: G01N 33/68, G01N 27/62

(54) **METHOD FOR ANALYZING NEUROGRANIN-RELATED PEPTIDE**

(30) Priority: 14.05.2021 JP 2021082340
(71) Applicant: Shimadzu Corporation, Nakagyo-ku, Kyoto-shi, Kyoto 604-8511 (JP)
(72) Inventor: KANEKO, Naoki, Kyoto-shi, Kyoto 604-8511 (JP)
(74) Representative: Müller-Boré & Partner Patentanwälte PartG mbB
(86) International application number: PCT/JP2022/014578
(87) International publication number: WO 2022/239537

(57) **Abstract**

A method for analyzing a neurogranin-related peptide contained in a biological sample includes, in the stated order: a binding step of bringing the biological sample into contact with a carrier in a binding solution to obtain a bound body in which the neurogranin-related peptide is bound to the carrier; a washing step of washing the bound body using a washing solution; an elution step of bringing the bound body into contact with an acidic solution to obtain an eluate in which the neurogranin-related peptide is eluted in the acidic solution; and a detection step of detecting the neurogranin-related peptide in the eluate by mass spectrometry. Both the binding solution and the washing solution contain a surfactant, and the number of carbon atoms of a hydrophobic group of each surfactant is 9 or more and 11 or less.

## Description

### TECHNICAL FIELD

The present invention relates to a method for analyzing a neurogranin-related peptide.

### BACKGROUND ART

Alzheimer's disease is the main cause of dementia, those suffered from Alzheimer's disease have increased more and more in recent years, and research on Alzheimer's disease has become even more important. In the development of Alzheimer's disease, an Aβ-related peptide such as amyloid β (AB) generated by cleavage of amyloid precursor protein (APP) is deeply involved. It has been reported that a plurality of Aβ-related peptides in blood are detected by combining immunoprecipitation method and mass spectrometry, and the detected specific Aβ-related peptide ratio is promising as a blood biomarker of amyloid accumulation in the brain (Non-Patent Documents 1 and 2 and Patent Documents 1 to 3).

Alzheimer's disease requires various biomarkers to capture disease progression of Alzheimer's disease, and there is a demand for biomarkers to reflect each process of tau accumulation and neurodegeneration in addition to amyloid accumulation. Among them, neurogranin is one of biomarkers of neurodegeneration, and is reported to increase in cerebrospinal fluid (CSF) of Alzheimer's disease patients (Non-Patent Documents 3 and 4). It has also been reported that fragmentation of neurogranin is promoted to generate fragment peptides in the brain of Alzheimer's disease patients (Non-Patent Document 5). Therefore, mass spectrometry of neurogranin or a fragment peptide thereof (neurogranin-related peptide) is expected as a means for capturing neurodegeneration.

Incidentally, in order to analyze a neurogranin-related peptide in cerebrospinal fluid, it is necessary to collect cerebrospinal fluid, which is not preferable in terms of invasiveness. Therefore, it is desired to perform analysis in blood which can be collected in a general test and is minimally invasive.

However, since the amount of the neurogranin-related peptide present in blood is very small, the neurogranin-related peptide cannot be sufficiently detected by conventional mass spectrometry. For example, it has been reported that neurogranin or a fragment peptide thereof could be detected by a method combining an immunoprecipitation method and mass spectrometry (Non-Patent Document 6).

### PRIOR ART DOCUMENTS

### PATENT DOCUMENTS

Patent Document 1: WO 2015/178398
Patent Document 2: WO 2017/47529
Patent Document 3: JP 2017-20980 A

### NON-PATENT DOCUMENTS

Non-Patent Document 1: Kaneko N, Nakamura A, Washimi Y, Kato T, Sakurai T, Arahata Y, Bundo M, Takeda A, Niida S, Ito K, Toba K, Tanaka K, Yanagisawa K. : Novel plasma biomarker surrogating cerebral amyloid deposition. Proc Jpn Acad Ser B Phys Biol Sci. 2014; 90 (9): 353-364.
Non-Patent Document 2: Nakamura A, Kaneko N, Villemagne VL, Kato T, Doecke J, Dore V, Fowler C, Li QX, Martins R, Rowe C, Tomita T, Matsuzaki K, Ishii K, Ishii K, Arahata Y, Iwamoto S, Ito K, Tanaka K, Masters CL, Yanagisawa K. : High performance plasma amyloid-β biomarkers for Alzheimer's disease. Nature. 2018; 554 (7691): 249-254.
Non-Patent Document 3: Portelius E, Olsson B, Hoglund K, Cullen NC, Kvartsberg H, Andreasson U, Zetterberg H, Sandelius A, Shaw LM, Lee VMY, Irwin DJ, Grossman M, Weintraub D, Chen-Plotkin A, Wolk DA, McCluskey L, Elman L, McBride J, Toledo JB, Trojanowski JQ, Blennow K. : Cerebrospinal fluid neurogranin concentration in neurodegeneration: relation to clinical phenotypes and neuropathology. Acta Neuropathol. 2018 ; 136 (3): 363-376.
Non-Patent Document 4: Thorsell A, Bjerke M, Gobom J, Brunhage E, Vanmechelen E, Andreasen N, Hansson O, Minthon L, Zetterberg H, Blennow K. : Neurogranin in cerebrospinal fluid as a marker of synaptic degeneration in Alzheimer's disease. Brain Res. 2010; 1362:13-22.
Non-Patent Document 5: Kvartsberg H, Lashley T, Murray CE, Brinkmalm G, Cullen NC, Hoglund K, Zetterberg H, Blennow K, Portelius E. : The intact postsynaptic protein neurogranin is reduced in brain tissue from patients with familial and sporadic Alzheimer's disease. Acta Neuropathol. 2019; 137 (1): 89-102.
Non-Patent Document 6: Kvartsberg H, Portelius E, Andreasson U, Brinkmalm G, Hellwig K, Lelental N, Kornhuber J, Hansson O, Minthon L, Spitzer P, Maler JM, Zetterberg H, Blennow K, Lewczuk P. : Characterization of the postsynaptic protein neurogranin in paired cerebrospinal fluid and plasma samples from Alzheimer's disease patients and healthy controls. Alzheimers Res Ther. 2015; 7 (1): 40.

### SUMMARY OF THE INVENTION

### PROBLEMS TO BE SOLVED BY THE INVENTION

However, even when the analysis method of Non-Patent Document 6 is performed and even when the analysis method of the Aβ-related peptide described in Non-Patent Document 2 is diverted to the neurogranin-related peptide, the analysis sensitivity is insufficient and has not reached the level of practical use. Therefore, in order to analyze neurogranin or a fragment peptide thereof in blood, further improvement in sensitivity is required.

An object of the present invention is to analyze a neurogranin-related peptide with high sensitivity.

### MEANS FOR SOLVING THE PROBLEMS

An analysis method of a first aspect of the present invention is a method for analyzing a neurogranin-related peptide contained in a biological sample, the analysis method including, in the stated order: a first binding step of bringing the biological sample into contact with a first carrier in a binding solution to obtain a first bound body in which the neurogranin-related peptide is bound to the first carrier; a first washing step of washing the first bound body using a first washing solution; a first elution step of bringing the first bound body into contact with a first acidic solution to obtain a first eluate in which the neurogranin-related peptide is eluted in the first acidic solution; a neutralization step of mixing the first eluate with a neutral buffer solution to obtain a purified solution; a second binding step of bringing the purified solution into contact with a second carrier to obtain a second bound body in which the neurogranin-related peptide is bound to the second carrier; a second washing step of washing the second bound body using a second washing solution; a second elution step of bringing the second bound body into contact with a second acidic solution to obtain a second eluate in which the neurogranin-related peptide is eluted in the second acidic solution; and a detection step of detecting the neurogranin-related peptide in the second eluate by mass spectrometry, in which all of the binding solution, the first washing solution, the neutral buffer solution, and the second washing solution contain a surfactant, and the number of carbon atoms of a hydrophobic group of each surfactant is 9 or more and 11 or less.

An analysis method of a second aspect of the present invention is a method for analyzing a neurogranin-related peptide contained in a biological sample, the analysis method including, in the stated order: a binding step of bringing the biological sample into contact with a carrier in a binding solution to obtain a bound body in which the neurogranin-related peptide is bound to the carrier; a washing step of washing the bound body using a washing solution; an elution step of bringing the bound body into contact with an acidic solution to obtain an eluate in which the neurogranin-related peptide is eluted in the acidic solution; and a detection step of detecting the neurogranin-related peptide in the eluate by mass spectrometry, in which both the binding solution and the washing solution contain a surfactant, and the number of carbon atoms of a hydrophobic group of each surfactant is 9 or more and 11 or less.

### EFFECTS OF THE INVENTION

According to the first and second aspects of the present invention, a neurogranin-related peptide can be analyzed with high sensitivity.

### BRIEF DESCRIPTION OF THE DRAWINGS

FIG. 1 shows a mass spectrum of an Ng-related peptide detected by a MALDI-TOF/MS apparatus in Reference Example 1.
FIG. 2 is a graph showing a relative ratio of sensitivity (S/N) of each Example and each Comparative Example to sensitivity (S/N) of Reference Example 1 in SIL-Ng50-78.
FIG. 3 is a graph showing a relative ratio of sensitivity (S/N) of each Example and each Comparative Example to sensitivity (S/N) of Reference Example 1 in Ng43-75.

### MODES FOR CARRYING OUT THE INVENTION

### 1. First aspect

An analysis method of a first aspect is a method for analyzing a neurogranin-related peptide in a biological sample, the analysis method including, in the stated order: a first binding step, a first washing step, a first elution step, a neutralization step, a second binding step, a second washing step, a second elution step, and a detection step. Hereinafter, each step will be described in detail.

The "neurogranin-related peptide" (hereinafter, abbreviated as "Ng-related peptide") includes neurogranin and a fragment peptide obtained by fragmenting neurogranin. Examples of the fragment peptide include Ng50-78 (SEQ ID NO: 1) and Ng43-75 (SEQ ID NO: 2).

### (First binding step)

In the first binding step, a biological sample is brought into contact with a first carrier in a binding liquid. For example, the binding solution, the biological sample, and the first carrier are mixed in an appropriate order. As a result, the Ng-related peptide in the biological sample is bound to the first carrier to obtain a first bound body.

Examples of the biological sample include body fluids such as blood, cerebrospinal fluid, urine, body secretion, saliva, and sputum; and feces. The blood includes whole blood, plasma, serum, and the like. The blood may be obtained by subjecting whole blood collected from an individual to a treatment such as centrifugation or cryopreservation. In the present analysis method, the biological sample is preferably blood. Blood is less invasive than cerebral bone marrow fluid, is a target sample for screening in a medical examination or the like, and is easily available.

The binding solution contains a surfactant. The binding solution is preferably a neutral buffer solution containing a surfactant.

Examples of the neutral buffer solution include a Tris buffer solution, a phosphate buffer solution, a HEPES buffer solution, and an ammonium acetate buffer solution. The pH of the neutral buffer solution is, for example, pH 6.0 or more and preferably 6.5 or more, and is, for example, 8.5 or less and preferably 8.0 or less.

In the surfactant contained in the binding solution, the number of carbon atoms of a hydrophobic group of the surfactant is 9 or more and 11 or less. That is, the surfactant contained in the binding solution consists of a surfactant which has a hydrophobic group having 9 to 11 carbon atoms. The number of carbon atoms of the hydrophobic group is more preferably 11. By containing a surfactant having such a carbon number, non-specific adsorption to the first bound body can be suppressed.

From the viewpoint of suppression of protein denaturation, ease of removal, reduction of ionization interference in mass spectrometry, and the like, a neutral surfactant is preferable. Examples of such a neutral surfactant include a surfactant having maltose in the hydrophilic portion, a surfactant having trehalose in the hydrophilic portion, and a surfactant having glucose in the hydrophilic portion.

Examples of the surfactant which has a hydrophobic group having 9 to 11 carbon atoms and has maltose include n-nonyl-β-D-maltoside (NM), n-nonyl-β-D-thiomaltoside (NTM), n-decyl-β-D-maltoside (DM), and n-undecyl-β-D-maltoside (UDM).

Examples of the surfactant which has a hydrophobic group having 9 to 11 carbon atoms and has trehalose include α-D-glucopyranosyl α-D-glucopyranoside monodecanoate (trehalose C10).

Examples of the surfactant which has a hydrophobic group having 9 to 11 carbon atoms and has glucose include n-decyl-β-D-glucoside.

These surfactants can be used singly or in combination of two or more kinds thereof.

The critical micelle concentration (cmc) of the surfactant is, for example, 0.2 mmol/L or more and preferably 0.4 mmol/L or more, and is, for example, 10 mmol/L or less and preferably 5 mmol/L or less. The critical micelle concentration is, for example, 0.01% or more and preferably 0.02% or more, and is, for example, 0.5% or less and preferably 0.3% or less. When the critical micelle concentration is in the above range, the analysis sensitivity of the Ng-related peptide can be improved.

The surfactant concentration in the binding solution is, for example, 0.02% (w/v) or more and preferably 0.2% (w/v) or more, and is, for example, 10% (w/v) or less and preferably 3% (w/v) or less. The surfactant concentration is, for example, 2 times or more and 20 times or less the critical micelle concentration. When the surfactant concentration is in the above range, micelles are sufficiently formed, so that the effect of the surfactant can be reliably achieved.

The first carrier may be any carrier to which the Ng-related peptide can be bound, and examples thereof include an antibody-immobilizing carrier.

The antibody immobilized on the first carrier is an antibody having an antigen binding site capable of recognizing an Ng-related peptide (anti-Ng-related peptide antibody), and examples thereof include an immunoglobulin having an antigen binding site capable of recognizing an Ng-related peptide, or a fragment thereof.

Examples of the immunoglobulin include IgG (IgG1, IgG2, IgG3, IgG4), IgM, IgA, IgY, IgD, and IgE. Examples of the immunoglobulin fragment include F(ab')2, F(ab'), F(ab), Fd, Fv, L chain, and H chain. More specific examples thereof include clones NG2, NG7, and EPR21152 and fragments thereof. The antibody may be either a monoclonal antibody or a polyclonal antibody.

Examples of the material for the first carrier include agarose, sepharose, dextran, silica gel, polyacrylamide, polystyrene, polyethylene, polypropylene, polyester, polyacrylonitrile, a (meth)acrylic acid-based polymer, a fluororesin, a metal complex resin, glass, a metal, and a magnetic body.

The shape of the first carrier may be any shape such as a spherical shape (including a bead shape), a plate shape, a needle shape, and an irregular shape, and may be a flow path wall or the like in a microdevice.

Before the first binding step, a pretreatment of removing an antibody such as IgG or IgM, or the like may be performed as necessary.

### (First washing step)

In the first washing step, after the first binding step, the first bound body is washed using a first washing solution.

The first washing solution contains a surfactant. The first washing solution is preferably a neutral buffer solution containing a surfactant.

The number of carbon atoms of the hydrophobic group of the surfactant contained in the first washing solution is 9 or more and 11 or less and most preferably 11. That is, the surfactant contained in the first washing solution consists of a surfactant which has a hydrophobic group having 9 to 11 carbon atoms. This makes it possible to effectively remove highly hydrophobic undesirable components (such as blood proteins, lipids, and glycolipids).

Examples of the neutral buffer solution and the surfactant in the first washing solution include those similar to the neutral buffer solution and the surfactant exemplified in the binding solution.

The surfactant concentration in the first washing solution is, for example, 0.01% (w/v) or more and preferably 0.1% (w/v) or more, and is, for example, 5% (w/v) or less and preferably 2% (w/v) or less. The surfactant concentration is, for example, equal to or more than the critical micelle concentration and 10 times or less the critical micelle concentration. When the surfactant concentration is in the above range, micelles are sufficiently formed, so that the effect of the surfactant can be reliably achieved.

As a washing method, a known method may be employed, and washing is preferably performed a plurality of times. For example, the bound body is washed using a neutral buffer solution containing a surfactant, and subsequently, washed using a neutral buffer solution not containing a surfactant.

As the neutral buffer solution not containing a surfactant, a neutral buffer solution similar to the neutral buffer solution exemplified in the binding solution can be used. This makes it possible to suppress foaming caused by the surfactant remaining in the first bound body.

As the washing method, a general method may be employed, and examples thereof include a method of stirring a carrier in a washing solution and a method of spraying a washing solution from a washing nozzle. After washing with these neutral buffer solutions, washing with water may be further performed as necessary.

### (First elution step)

In the first elution step, after the first washing step, the first bound body is brought into contact with a first acidic solution. As a result, the Ng-related peptide is dissociated from the first bound body, and the neurogranin is eluted into the first acidic solution. As a result, a first eluate containing an Ng-related peptide is obtained.

Examples of the first acidic solution include acidic aqueous solutions such as a glycine buffer solution and hydrochloric acid, and a glycine buffer solution is preferable. The pH of the first acidic solution is, for example, 3.5 or less preferably 3.0 or less, and is, for example, 0.5 or more and preferably 1.0 or more.

The first acidic solution preferably contains a surfactant. The number of carbon atoms of the hydrophobic group of the surfactant contained in the first acidic solution is 9 or more and 11 or less and most preferably 11. That is, the surfactant contained in the first acidic solution consists of a surfactant which has a hydrophobic group having 9 to 11 carbon atoms. This makes it possible to more reliably dissociate the Ng-related peptide from the first bound body. The eluted Ng-related peptide is inhibited from adhering to a container such as a test tube or a microplate. Therefore, the recovery rate of the Ng-related peptide can be reliably improved.

Examples of the surfactant in the first acidic solution include those similar to the surfactant exemplified in the binding solution. The surfactant concentration in the first acidic solution is the same as the surfactant concentration in the first washing solution.

### (Neutralization step)

In the neutralization step, after the first elution step, the first eluate is mixed with a neutral buffer solution. As a result, the first eluate is neutralized to obtain a purified solution containing an Ng-related peptide.

The neutral buffer solution used in the neutralization step may contain a surfactant. In particular, when the first acidic solution (and the first eluate) does not contain a surfactant, the neutral buffering agent preferably contains a surfactant. The number of carbon atoms of the hydrophobic group of the surfactant contained in the neutral buffer solution is 9 or more and 11 or less and most preferably 11. That is, the surfactant contained in the purified solution consists of a surfactant which has a hydrophobic group having 9 to 11 carbon atoms. As a result, non-specific adsorption to the second bound body in the second binding step can be suppressed.

Examples of the neutral buffer solution and the surfactant used in the neutralization step include those similar to the neutral buffer solution and the surfactant exemplified in the binding solution. The surfactant concentration in the neutral buffer solution is the same as the surfactant concentration in the first binding solution.

The pH of the purified solution is neutral, and is, for example, pH 6.0 or more and preferably 6.5 or more, and is, for example, 8.5 or less and preferably 8.0 or less. This makes it possible to improve the binding efficiency in the second binding step.

### (Second binding step)

In the second binding step, after the neutralization step, the purified solution is brought into contact with the second carrier. As a result, the Ng-related peptide of the purified solution is bound to the second carrier to obtain a second bound body.

The second carrier is preferably an antibody-immobilizing carrier, and specific examples thereof include those similar to the antibody-immobilizing carrier exemplified in the first carrier.

### (Second washing step)

In the second washing step, after the second binding step, the second bound body is washed using a second washing solution.

The second washing solution contains a surfactant. The second washing solution is preferably a neutral buffer solution containing a surfactant.

The number of carbon atoms of the hydrophobic group of the surfactant contained in the second washing solution is 9 or more and 11 or less and most preferably 11. That is, the surfactant contained in the second washing solution consists of a surfactant which has a hydrophobic group having 9 to 11 carbon atoms. This makes it possible to effectively remove, for example, highly hydrophobic undesirable components (such as blood proteins, lipids, and glycolipids).

Examples of the neutral buffer solution and the surfactant used in the second washing solution include those similar to the neutral buffer solution and the surfactant exemplified in the binding solution. The surfactant concentration in the second washing solution is the same as the surfactant concentration in the first washing solution.

As the washing method, a known method may be employed, and specifically, a method similar to the washing method exemplified in the first washing step may be performed.

### (Second elution step)

In the second elution step, after the second washing step, the second bound body is brought into contact with a second acidic solution. As a result, the Ng-related peptide is dissociated from the second bound body, and the Ng-related peptide is eluted into the second acidic solution. As a result, a second eluate containing an Ng-related peptide is obtained.

Examples of the second acidic solution include those similar to the first acidic solution exemplified in the first elution step, and hydrochloric acid is preferable.

The second acidic solution preferably contains a volatile organic solvent. This makes it possible to efficiently dissociate the Ng-related peptide from the second bound body so as to elute the Ng-related peptide in the second acidic solution, and to improve the recovery rate of the Ng-related peptide.

Examples of the volatile organic solvent include organic solvents miscible with water in an arbitrary ratio, and examples thereof include acetonitrile, methanol, ethanol, acetone, toluene, isopropanol, hexane, butanol, cyclohexane, ethylene glycol, benzene, chloroform, acetaldehyde, triethylamine, phenol, naphthalene, formaldehyde, tetrahydrofuran, and ethyl acetate. Acetonitrile, methanol, ethanol, acetone, isopropanol, and the like are preferable. These organic solvents can be used singly or in combination of two or more kinds thereof.

The volatile organic solvent concentration in the second acidic solution is, for example, 10% (v/v) or more and preferably 25% (v/v) or more, and is, for example, 90% (v/v) or less and preferably 80% (v/v) or less. When the concentration is within the above range, the Ng-related peptide can be efficiently dissociated from the second carrier, and the sensitivity (S/N ratio) at the time of mass spectrometry can be improved.

The second acidic solution preferably further contains an amino acid such as methionine. As a result, the oxidation of the Ng-related peptide can be reduced until the analysis is started after the Ng-related peptide is placed in a mass spectrometer, and the analysis accuracy can be improved. The amino acid concentration in the second acidic solution is, for example, 0.01 mM or more and preferably 0.05 mM or more, and is, for example, 5 mM or less and preferably 1 mM or less.

### (Analysis step)

In the analysis step, after the second elution step, the Ng-related peptide contained in the second eluate is detected by mass spectrometry.

Examples of the mass spectrometry include MALDI (Matrix Assisted Laser Desorption/Ionization), ESI (Electrospray ionization), and APCI (Atmospheric Pressure Chemical Ionization). MALDI is preferable from the viewpoint that the measurement can be performed without using liquid chromatography, so that loss such as adsorption can be reduced, and the Ng-related peptide can be reliably ionized even when some impurities coexist.

For detection of MALDI, for example, operation may be performed according to a conventional method using a MALDI-TOF (matrix assisted laser desorption/ionization-time of flight)-type mass spectrometer, a MALDI-IT (matrix assisted laser desorption/ionization-ion trap)-type mass spectrometer, a MALDI-IT-TOF (matrix assisted laser desorption/ionization-ion trap-time of flight)-type mass spectrometer, a MALDI-FTICR (matrix assisted laser desorption/ionization-Fourier transform ion cyclotron resonance)-type mass spectrometer, or the like.

Upon detection with MALDI, the matrix is placed, for example, by dropping a matrix-containing solution onto a MALDI plate and drying the matrix-containing solution.

Examples of the matrix include α-cyano-4-hydroxycinnamic acid (CHCA), 2,5-dihydroxybenzoic acid, sinapic acid, and 3-aminoquinoline. These matrices can be used singly or in combination of two or more kinds thereof.

Examples of the solvent contained in the matrix include acetonitrile, trifluoroacetic acid, methanol, ethanol, and water. These solvents can be used singly or in combination of two or more kinds thereof.

The matrix concentration in the matrix-containing solvent is, for example, 0.1 mg/mL or more and preferably 0.5 mg/mL or more, and is, for example, 50 mg/mL or less and preferably 10 mg/mL or less.

Preferably, a matrix additive is used in combination with the matrix. Examples of the matrix additive include a phosphonic acid group-containing compound and an ammonium salt, and from the viewpoint that an adverse effect on the background due to the remaining of the washing solution, a phosphonic acid group-containing compound is preferable. Examples of the phosphonic acid group-containing compound include phosphonic acid, methylphosphonic acid, phenylphosphonic acid, 1-naphthylmethylphosphonic acid, methylenediphosphonic acid (MDPNA), ethylene diphosphonic acid, ethane-1-hydroxy-1,1-diphosphonic acid, nitrilotriphosphonic acid, and ethylene diaminotetraphosphonic acid.

The matrix additive concentration in the matrix-containing solvent is, for example, 0.01% (w/v) or more and preferably 0.1% (w/v) or more, and is, for example, 10% (w/v) or less and preferably 1% (w/v) or less.

As a result, the Ng-related peptide contained in the second eluate (and the biological sample) can be measured. In this analysis method, in particular, any of the binding solution used in the first binding solution, the first washing solution used in the first washing step, the neutral buffer solution used in the neutralization step, and the second washing solution used in the second washing step contains a specific surfactant in which the number of carbon atoms of the hydrophobic group is 9 or more and 11 or less, whereby the binding efficiency of the Ng-related peptide to the carrier in the binding step, the recovery rate of the Ng-related peptide in the elution step, and the like can be improved to high levels, and as a result, the Ng-related peptide can be analyzed with very high sensitivity (S/N) as compared with the conventional analysis method.

### 2. Second aspect

In the analysis method of the first aspect, an immunoprecipitation method (affinity purification) is performed twice, but for example, the immunoprecipitation method may be performed once. In this case, the analysis method of the second aspect includes a first binding step (binding step), a first washing step (washing step), a second elution step (elution step), and a detection step. Each step is the same as in the first aspect. The second aspect also has the same effect as in the first aspect. From the viewpoint that the Ng-related peptide can be reliably analyzed even when the abundance of the Ng-related peptide in the biological sample is even smaller, the first aspect is preferable.

### 3. Aspects

It is understood by those skilled in the art that the plurality of exemplary embodiments described above are specific examples of the following aspects.

(Item 1) An analysis method according to an aspect is a method for analyzing a neurogranin-related peptide contained in a biological sample, the analysis method including, in the stated order: a binding step of bringing the biological sample into contact with a carrier in a binding solution to obtain a bound body in which the neurogranin-related peptide is bound to the carrier; a washing step of washing the bound body using a washing solution; an elution step of bringing the bound body into contact with an acidic solution to obtain an eluate in which the neurogranin-related peptide is eluted in the acidic solution; and a detection step of detecting the neurogranin-related peptide in the eluate by mass spectrometry, in which both the binding solution and the washing solution may contain a surfactant, and the number of carbon atoms of a hydrophobic group of each surfactant may be 9 or more and 11 or less.

(Item 2) In the analysis method according to Item 1, the acidic solution may contain an organic solvent.

(Item 3) In the analysis method according to Item 1 or 2, the biological sample may be blood.

(Item 4) In the analysis method according to any one of Items 1 to 3, the mass spectrometry may be matrix assisted laser desorption/ionization.

(Item 5) An analysis method according to an aspect is a method for analyzing a neurogranin-related peptide contained in a biological sample, the analysis method including, in the stated order: a first binding step of bringing the biological sample into contact with a first carrier in a binding solution to obtain a first bound body in which the neurogranin-related peptide is bound to the first carrier; a first washing step of washing the first bound body using a first washing solution; a first elution step of bringing the first bound body into contact with a first acidic solution to obtain a first eluate in which the neurogranin-related peptide is eluted in the first acidic solution; a neutralization step of mixing the first eluate with a neutral buffer solution to obtain a purified solution; a second binding step of bringing the purified solution into contact with a second carrier to obtain a second bound body in which the neurogranin-related peptide is bound to the second carrier; a second washing step of washing the second bound body using a second washing solution; a second elution step of bringing the second bound body into contact with a second acidic solution to obtain a second eluate in which the neurogranin-related peptide is eluted in the second acidic solution; and a detection step of detecting the neurogranin-related peptide in the second eluate by mass spectrometry, in which all of the binding solution, the first washing solution, and the second washing solution may contain a surfactant, and the number of carbon atoms of a hydrophobic group of each surfactant may be 9 or more and 11 or less.

(Item 6) In the analysis method according to Item 5, the second acidic solution may contain an organic solvent.

(Item 7) In the analysis method according to Item 5 or 6, the first acidic solution may contain a surfactant, and the number of carbon atoms of a hydrophobic group of the surfactant may be 9 or more and 11 or less.

(Item 8) In the analysis method according to any one of Items 5 to 7, the biological sample may be blood.

(Item 9) In the analysis method according to any one of Items 5 to 8, the mass spectrometry may be matrix assisted laser desorption/ionization.

### EXAMPLES

The present invention will be described in detail with reference to Examples and Comparative Examples; however, the scope of the present invention is not limited thereto.

### <Reference Example 1>

As Reference Example 1, the Ng-related peptide was analyzed as follows with reference to the mass spectrometry method of amyloid β described in Non-Patent Document 2.

### (First binding step, first washing step, and first elution step)

A clone NG2 (BioLegend, Inc.) of an anti-Ng antibody (IgG1) having 52-63 residues of human Neurogranin (Ng) as an epitope was prepared. To 100 pg of an anti-Ng antibody, 4.95 mg of magnetic beads (Dynabeads M-270 Epoxy) were added in an immobilization buffer solution (0.1 M phosphate buffer solution containing 1.5 M ammonium sulfate; pH 7.4) at 37°C for 16 to 24 hours. As a result, antibody beads were produced.

Human plasma was spiked so that stable isotope-labeled Ng50-78 (SIL-Ng50-78) was 300 pM. The plasma (250 pL) was mixed with 250 pL of a binding buffer solution (surfactant [0.2% DDM and 0.2% NTM], 800 mM GlcNAc, 100 mM Tris-HCl, 300 mM NaCl; pH 7.4) and allowed to stand still on ice for 5 to 60 minutes. The plasma was mixed with antibody beads and shaken on ice for 1 hour. Thereafter, the antibody beads were washed 3 times with 100 pL of a first washing buffer solution (surfactant [0.1% DDM and 0.1% NTM], 50 mM Tris-HCl, 150 mM NaCl; pH 7.4) and 2 times with 50 µL of a 50 mM ammonium acetate buffer solution. The antibody beads were then brought into contact with the first acidic solution (50 mM glycine buffer solution containing a surfactant [0.1% DDM]; pH 2.8) to elute the Ng-related peptide in the first acidic solution. As a result, a first eluate containing an Ng-related peptide was obtained.

### (Neutralization step)

The first eluate was mixed with a neutral buffer solution (surfactant [0.2% DDM], 800 mM GlcNAc, 300 mM Tris-HCl, 300 mM NaCl; pH 7.4) to obtain a purified solution.

### (Second binding step, second washing step, and second elution step)

The purified solution was mixed with antibody beads and shaken on ice for 1 hour. Thereafter, the antibody beads were washed 5 times with 50 pL of a second washing buffer solution (surfactant [0.1% DDM], 150 mM Tris-HCl, 150 mM NaCl; pH 7.4), 2 times with 50 pL of a 50 mM ammonium acetate buffer solution, and once with 30 µL of water. The antibody beads were then brought into contact with the second acidic solution (70% (v/v) acetonitrile aqueous solution containing 5 mM hydrochloric acid and 0.1 mM methionine; pH 2.3) to elute the Ng-related peptide in the second acidic solution. As a result, a second eluate containing an Ng-related peptide was obtained.

### (Analysis step)

As a mass spectrometer, a MALDI-TOF MS apparatus (manufactured by SHIMADZU CORPORATION) was used. A0.5 mg/mL CHCA/0.2% (w/v) MDPNAmatrix solution was prepared using α-cyano-4-hydroxycinnamic acid (CHCA) as a matrix for Linear TOF, methylenediphosphonic acid (MDPNA) as a matrix additive, and acetonitrile as a solvent. To 4 wells of a MALDI plate (µFocus MALDI plate 900 pm (Hudson Surface Technology, Inc., Fort Lee, NJ)), 0.5 pL each of the matrix solution was added dropwise and dried. The second eluate was added dropwise to these 4 wells and placed.

MALDI-TOF MS was then activated to detect an Ng-related peptide. As a setting condition, mass spectrum data was acquired by Linear TOF in a positive ion mode using AXIMA Performance (Shimadzu/KRATOS, Manchester, UK). For 1 well, 400 spots and 16000 shots were accumulated. The m/z value of Linear TOF was expressed as a peak average mass. The m/z value was calibrated using human angiotensin II and human ACTH fragment 18-39, bovine insulin oxidized beta-chain, bovine insulin, cytochrome c as external standards.

### <Examples 1 to 3>

The analysis method was performed in the same manner as in Reference Example 1, except that the surfactant contained in each solution and the concentration thereof were changed to the surfactant and the concentration thereof described in Table 1.

### <Comparative Examples 1 to 3>

The analysis method was performed in the same manner as in Reference Example 1, except that the surfactant contained in each solution and the concentration thereof were changed to the surfactant and the concentration thereof described in Table 1.

**[Table 1]**

| | Reference Example 1 | Comparative Example 1 | Comparative Example 2 | Example 1 | Example 2 | Example 3 | Comparative Example 3 |
|---|---|---|---|---|---|---|---|
| Binding buffer solution | D D M (0.2%), N T M (0.2%) | T C12 (0.1%) | D D M (0.1%) | U D M (0.4%) | D M (1.0%) | N T M (2.0%) | O G (10.0%) |
| First washing buffer solution | D D M (0.1%), N T M (0.1%) | T C12 (0.05%) | D D M (0.05%) | U D M (0.2%) | D M (0.5%) | N T M (1.0%) | O G (5.0%) |
| First acidic solution | D D M (0.1%) | T C12 (0.05%) | D D M (0.05%) | U D M (0.2%) | D M (0.5%) | N T M (10%) | O G (5.0%) |
| Neutral buffer solution of neutral ization step | D D M (0.2%) | T C12 (0.1%) | D D M (0.1%) | U D M (0.4%) | D M (1.0%) | N T M (2.0%) | O G (10.0%) |
| Second washing buffer solution | D D M (0.1%) | T C12 (0.05%) | D D M (0.05%) | U D M (0.2%) | D M (0.5%) | N T M (1.0%) | O G (5.0%) |

| | | | | | | | |
|---|---|---|---|---|---|---|---|
| In Table 1, % indicates % (w/v). | | | | | | | |

Abbreviations and their carbon numbers and critical micelle numbers in the table are shown below.
TO 12: Trehalose C12
DDM: n-Dodecyl-β-D-maltoside
UDM: n-Undecyl-β-D-maltoside
DM: n-Decyl-β-D-maltoside
NTM: n-Nonyl-β-D-thiomaltoside
OG: n-Octyl-β-D-glucoside

**[Table 2]**

| | TC12 | DDM | UDM | DM | NTM | OG |
|---|---|---|---|---|---|---|
| Number of carbon atoms of hydrophobic group | 12 | 12 | 11 | 10 | 9 | 8 |
| Critical micelle concentration : cmc(mmol/l) | 0.15 | 0.17 | 0.55 | 1.8 | 2.4 | 25 |
| Critical micelle concentration : cmc(%) | 0.0079 | 0.0087 | 0.0273 | 0.0869 | 0.1163 | 0.7309 |

### <Analysis result>

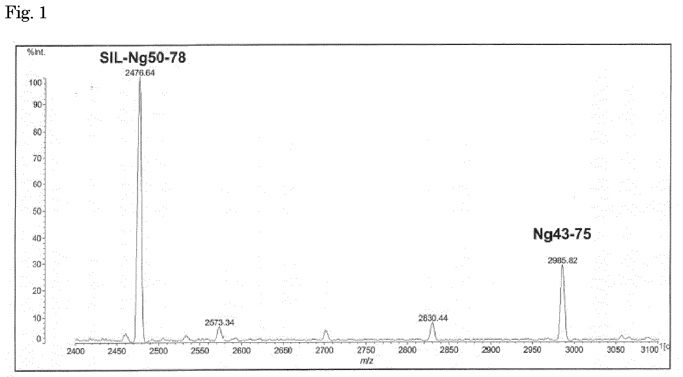

One (1 well) of the mass spectrum obtained in Reference Example 1 is shown in FIG. 1. Peaks corresponding to the masses of SIL-Ng50-78 and Ng43-75 were extracted from the detected peaks, and S/N of each peak displayed on analysis software (Shimadzu Biotach Launchpad: Shimadzu/KRATOS, Manchester, UK) of AXIMA Performance was used as an index of sensitivity. At this time, an average value in a mass spectrum of 4 wells was adopted as S/N. Table 3 shows the amino acid sequences of SIL-Ng50-78 and Ng43-75 and Theoretical average m/z. Ng43-75 is a plasma endogenous Ng peptide.

**[Table 3]**

| Name | Amino acid sequence | Theoretical average *m*/*z* |
|---|---|---|
| SIL-Ng50-78 | ERGRKGPGPGGPGGAGVARGGAGGGPSGD | 2476.68 |
| Ng43-75 | RKKIKSGERGRKGPGPGGPGGAGVARGGAGGGP | 2985.4 |

In each of Examples and Comparative Examples, S/N at each peak of SIL-Ng50-78 and Ng43-75 was calculated from the spectrum of 4 wells in the same manner as in Reference Example 1.

At each peak of SIL-Ng50-78 and Ng43-75, the relative ratio of S/N of Examples 1 to 3 to S/N of Reference Example 1 and the relative ratio of S/N of Comparative Examples 1 to 3 to S/N of Reference Example 1 were calculated.

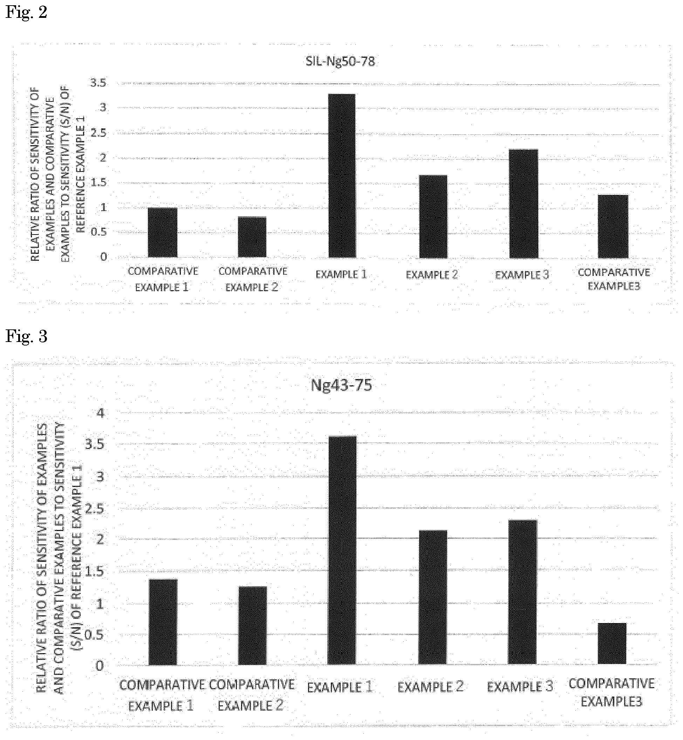

A series of analysis experiments including the above Reference Example, each Example, and each Comparative Example was repeatedly performed, and analysis data (the above relative ratio) of 4 to 5 times was acquired, and then an average value of these 4 to 5 times was calculated. The results thereof are shown in FIGS. 2 and 3.

As apparent from FIGS. 2 and 3, as compared with Reference Example 1, in Examples 1 to 3, improvement in sensitivity (S/N) by 1.5 times or more was confirmed, and in Example 1, improvement in sensitivity by 3 times or more was confirmed. As a result, in the present analysis method, the Ng fragment peptide can be detected from blood with very high sensitivity, and the Ng fragment peptide in blood can be reliably measured. On the other hand, in Comparative Examples 1 to 3, the sensitivity was equivalent to that in Reference Example 1.

## Claims

1. A method for analyzing a neurogranin-related peptide contained in a biological sample, the analysis method comprising, in the stated order:
a binding step of bringing the biological sample into contact with a carrier in a binding solution to obtain a bound body in which the neurogranin-related peptide is bound to the carrier;
a washing step of washing the bound body using a washing solution;
an elution step of bringing the bound body into contact with an acidic solution to obtain an eluate in which the neurogranin-related peptide is eluted in the acidic solution; and
a detection step of detecting the neurogranin-related peptide in the eluate by mass spectrometry,
wherein both the binding solution and the washing solution contain a surfactant, and
the number of carbon atoms of a hydrophobic group of each surfactant is 9 or more and 11 or less.

2. The analysis method according to claim 1, wherein the acidic solution contains an organic solvent.

3. The analysis method according to claim 1, wherein the biological sample is blood.

4. The analysis method according to any one of claims 1 to 3, wherein the mass spectrometry is matrix assisted laser desorption/ionization.

5. A method for analyzing a neurogranin-related peptide contained in a biological sample, the analysis method comprising, in the stated order:
a first binding step of bringing the biological sample into contact with a first carrier in a binding solution to obtain a first bound body in which the neurogranin-related peptide is bound to the first carrier;
a first washing step of washing the first bound body using a first washing solution;
a first elution step of bringing the first bound body into contact with a first acidic solution to obtain a first eluate in which the neurogranin-related peptide is eluted in the first acidic solution;
a neutralization step of mixing the first eluate with a neutral buffer solution to obtain a purified solution;
a second binding step of bringing the purified solution into contact with a second carrier to obtain a second bound body in which the neurogranin-related peptide is bound to the second carrier;
a second washing step of washing the second bound body using a second washing solution;
a second elution step of bringing the second bound body into contact with a second acidic solution to obtain a second eluate in which the neurogranin-related peptide is eluted in the second acidic solution; and
a detection step of detecting the neurogranin-related peptide in the second eluate by mass spectrometry,
wherein all of the binding solution, the first washing solution, and the second washing solution contain a surfactant, and
the number of carbon atoms of a hydrophobic group of each surfactant is 9 or more and 11 or less.

6. The analysis method according to claim 5, wherein the second acidic solution contains an organic solvent.

7. The analysis method according to claim 5, wherein
the first acidic solution contains a surfactant, and
the number of carbon atoms of a hydrophobic group of the surfactant is 9 or more and 11 or less.

8. The analysis method according to claim 5, wherein the biological sample is blood.

9. The analysis method according to claim 5, wherein the mass spectrometry is matrix assisted laser desorption/ionization.
